# EUROPEAN PATENT APPLICATION

(11) **EP 3 366 136 A1**
(43) Date of publication of application: **29.08.2018**
(21) Application number: 16805478.1
(22) Date of filing: 21.10.2016
(51) Int. Cl.: A01N 31/06, A01N 31/16, A01N 63/04, A01P 1/00, A01P 3/00, A01P 5/00, A01P 7/04

(54) **NATURAL BROAD-SPECTRUM BIOCIDES**

(30) Priority: 23.10.2015 ES 201531527
(71) Applicant: Consejo Superior De Investigaciones Científicas, 28006 Madrid (ES); Universidad De La Laguna, 38200 Santa Cruz De Tenerife (ES); The Energy and Resources Institute (TERI), New Delhi 110003 (IN)
(72) Inventor: GONZÁLEZ COLOMA, Azucena, 28006 Madrid (ES); ANDRES YEVES, María Fe, 28006 Madrid (ES); DIAZ HERNANDEZ, Carmen Elisa, 38206 La Laguna (ES); REINA ARTILES, Matías, 38206 La Laguna (ES); LACRET PIMIENTA, Rodney, 28006 Madrid (ES); CABRERA PEREZ, Raimundo, 38206 La Laguna (ES); GIMENEZ MARIÑO, Cristina, 38206 La Laguna (ES); KAUSHIK, Nutan, New Delhi 110003 (IN)
(74) Representative: ZBM Patents - Zea, Barlocci & Markvardsen
(86) International application number: PCT/ES2016/070746
(87) International publication number: WO 2017/068223

(57) **Abstract**

In the field of agriculture, organisms that are harmful to vegetation are a major problem in relation to the productivity of harvests. The present invention relates to a fermentation product obtained from endophytic fungi of the *Stemphylium solani* family, which may comprise a novel compound of formula (I) and a second known compound of formula (II), and having biocidal activity simultaneously against more than one category of harmful organisms affecting plants, and which are preferably selected from insect-pests, fungi and nematodes. Both the fermentation product, and the compounds included in the scope of the invention, are used in the preparation of a broad-spectrum biocidal composition. Finally, the invention also includes the process for preparing of the fermentation product and the new compound of formula (I).

## Description

### FIELD OF THE INVENTION

The invention relates to the field of agriculture, and, in particular, to the field of biocides comprising substances produced or extracted from micro-organisms. A fermentation product with broad-spectrum biocidal activity and a process for its preparation is proposed. The fermentation product is characterised in that it comprises at least one novel compound of formula (I), which is also claimed, and a compound of formula (II), which give the fermentation product its characteristic biocidal activity. The invention also provides a particular strain of *Stemphylium solani* and biocidal compositions comprising the fermentation product, the compound of formula (I) and/or the strain of the invention. Finally, the invention also relates to the use of the fermentation product, the compound of formula (I) and/or the strain of the invention either directly or through a biocidal composition comprising thereof as biocides, and a broad-spectrum method for the control of organisms harmful to plants.

### BACKGROUND ART

Endophytic fungi are a polyphyletic group with a high degree of diversity that is functionally characterised by their ability to live in the plant tissues without causing any apparent damage. Colonisation by endophytic fungi can contribute to the host plant's adaptation to stress and in many cases the tolerance of the host plant to biotic stress has been correlated with the presence of natural fungal products (Aly et al. 2010. Fungal Diversity 41, 1-16).

The genus *Stemphylium* is composed of filamentous and saprophytic fungi of the hyphomycetes group, which are widely distributed around the world, associated with decaying vegetation. In agriculture, species of *Stemphylium* are responsible for diseases in many crops, to which they cause leaf damage, and are dispersed through the seeds.

Among the metabolites produced by the *Stemphylium* species of greater phytopathogenic importance which are: S*. botryosum,* S*. herbarum,* S*. alfalfae,* S. *sarciniforme,* five main compounds have been identified, stemphylin, stemphyloxin II, stemphyperilenol, stemphol and a related compound (Barash et al. 1975. Plant Physiol. 55, 646-651; Andersen et al. 1995. Mycol. Res. 99, 672-676; Solfrizzo et al. 1994. Nat. Toxins 2, 14-18). Furthermore, the production of taxol by a species of *Stemphylium* sp. isolated as a *Taxus baccata* endophyte has been described (Mirjalili et al. 2012. FEMS Microbiol. Lett. 328, 122-9).

Several species of *Stemphylium* have been isolated as endophytes from different plants, for example, in the endemic Australian plant *Eremophilia longifolia* (Zaferanloo et al. 2013. World J Microbiol. Biotechnol. 29, 335-345); in *Vitis vinifera* (Gonzalez and Tello. 2011. Fungal Diversity 47: 29-42); and even a strain of *S. solani* has been identified in *Arabidopsis thaliana* (Garcia et al. 2012. Fungal Diversity. DOI :10.1007/s13225-012-0219-0).

Moreover, from extracts of *Stemphylium globuliferum* from *Mentha pulegium* (Debbab et al. 2009. J. Nat. Prod. 72, 626-31), the compounds: alterporriol G and its isomer terporriol H, altersolanol, altersolanol L, stemphypyrone, 6-methylalaternin, macrosporin, altersolanol A, alterporriol E, alterporriol D, alterporriol A, alterporriol B, and altersolanol J have been identified.

Agriculture is constantly faced with serious problems related to the incidence of pests and attacks by pathogenic organisms with the consequent reduced productivity. Although traditionally the usual method for controlling harmful organisms affecting plants has been the application of synthetic chemicals, which in addition to their high economic price imply a serious environmental cost, the new regulatory environment, see, for example, both the national and European Regulation (EC) No. 1107/2009, has drastically limited the number of active materials and the availability of phytosanitary products for the control of diseases caused by these harmful organisms.

Within this scenario, the search for active, alternative and effective compounds, which have little persistence in the environment, which reduce the occurrence of cross-resistance, which do not have undesirable cytotoxic effects and which come from, for example, endophytic fungi, seems to be a good alternative. Additionally, it would be highly desirable that these compounds were of broad-spectrum activity, that is, were active against various harmful organisms simultaneously.

### SUMMARY OF THE INVENTION

The present invention provides, in a first aspect, a fermentation product comprising a compound of formula (I): 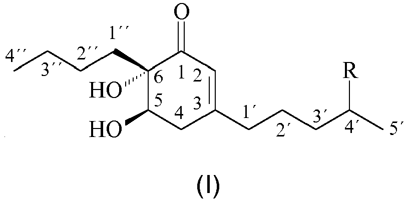 where R is selected from H and OH,
or an isomer, or a salt or a solvate thereof.

The present invention also provides a fermentation product of an endophytic fungus of the species *Stemphylium solani* with broad-spectrum biocidal activity, characterised in that it comprises a compound of formula (I): 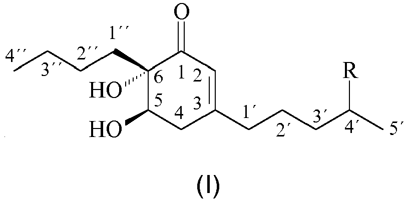 where R is selected from H and OH,
or an isomer, or a salt or a solvate thereof.

In a second aspect, the invention provides the novel compound of formula (I) 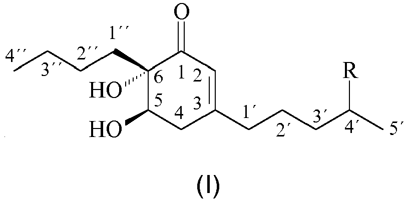 where R is selected from H and OH,
or an isomer, or a salt or a solvate thereof.

In a third aspect, the invention provides a process for preparing the fermentation product defined in the first aspect of the invention, comprising (a) a fermentation step comprising culturing the fermenting micro-organism in a suitable culture medium and under suitable conditions; and optionally (b) one or more extraction steps.

Preferably, the endophytic fungus used in the process for preparing is the Aa22 strain of *S*. *solani* with deposit number CECT 20941 (Spanish Type Culture Collection).

Although both the compounds of formula (I) of the invention and the compound of formula (II) 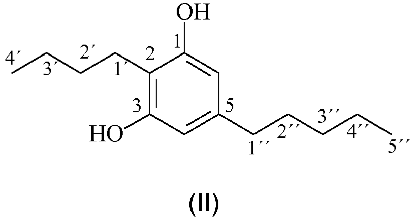 separately have broad-spectrum biocidal activity, the inventors of the present invention have found that the fermentation product resulting from the process of the third aspect of the invention and comprising the compound of formula (I) and, optionally, the compound of formula (II) has a demonstrably improved activity (see Examples 4 to 6).

Thus, in a fourth aspect, the invention provides a fermentation product, which is an extract comprising the compound of formula (I) as defined in the second aspect of the invention, obtainable by the process as defined in the third aspect of the invention.

In a fifth aspect, the present invention provides a process for preparing a compound of formula (I) as defined in the second aspect of the invention, comprising the step of isolating the compound of formula (I) from the fermentation product resulting from step (b), (i.1.), (i.2) or (c) defined below.

In a sixth aspect, the present invention provides an isolated strain of the endophytic fungi from the species *Stemphylium solani* deposited in the Spanish Type Culture Collection with deposit number CECT20941 or a mutant thereof that maintains the ability to produce the compound of formula (I).

As demonstrated below, the fermentation products of the invention as well as the compounds of formula (I) exhibit a broad-spectrum biocidal effect.

Therefore, in a seventh aspect, the present invention provides the use of a fermentation product as defined in the first aspect of the invention, or of the fermentation product as defined in the fourth aspect of the invention, or the compound of general formula (I) as defined in the second aspect of the invention, or the compound of formula (II) as defined below, or the strain of *Stemphylium solani* of the sixth aspect of the invention as a broad-spectrum biocidal agent.

In an eighth aspect, the invention is related to the use of the fermentation product, or of the compounds of formulas (I) or (II), alone or in combination, or of the strain *Stemphylium solani* of the invention to prepare a broad-spectrum biocidal composition.

In a ninth aspect, the invention provides a biocidal composition comprising the fermentation product as defined in the first aspect of the invention, or the fermentation product as defined in the fourth aspect of the invention, or the compound of formula (I) as defined in the second aspect of the invention, or the compound of formula (II) as defined below, or the strain of *Stemphylium solani* of the sixth aspect of the invention.

In a tenth aspect, the invention provides the use of the fermentation product of the invention, or of the compound of the invention, or of a compound of formula (II), either alone or in combination, or of the strain of *Stemphylium solani* of the invention to prepare a biocidal composition.

In an eleventh aspect, the invention is related to the use of the biocidal composition as a broad-spectrum biocidal agent to control at least one category of harmful organisms affecting plants, and which are selected from insect-pests, fungi, and nematodes, preferably all of them simultaneously.

In a final aspect, the invention provides a method for controlling harmful organisms affecting plants, hereinafter, method of control of the invention, comprising administering an effective dose of the fermentation product as defined in the first aspect of the invention, or of the fermentation product as defined in the fourth aspect of the invention, or of the compound of formula (I) as defined in the second aspect of the invention, or of the compound of formula (II) as defined below, or of the strain of *Stemphylium solani* as defined in the sixth aspect of the invention, or of the biocidal composition as defined in the ninth aspect of the invention.

### DETAILED DESCRIPTION OF THE INVENTION

The technical problem solved by the present invention is the search for new natural products that are effective biocides.

Surprisingly, the present inventors have identified a number of fermentation products, extracts and compounds having a broad-spectrum biocidal activity, allowing the simultaneous control of several harmful organisms affecting plants. Therefore, the present invention means an effective alternative to synthetic chemical biocides.

The present invention is based on the discovery of a fermentation product from an endophytic fungus of the species *Stemphylium solani,* which preferably is the Aa22 strain *S*. *solani* with deposit number CECT 20941, and which can simultaneously comprise at least one compound of formula (I) and one compound of formula (II), providing it with a surprising broad-spectrum biocidal activity against phytopathogenic fungi, insect-pests and the phytopathogenic nematode *Meloidogyne javanica* (see Examples 4 to 6).

The main technical advantages of the fermentation product, of its use for the control of harmful organisms affecting plants and of its process of preparation, are listed below:
- it is of broad-spectrum activity, simultaneously acting effectively against insect-pests, fungi and nematodes,
- it is obtained from natural sources, by simple and inexpensive procedures, and
- it can be obtained on large scale by fermentation of the fungus in bioreactors, with the possibility of adjusting the fermentation conditions to increase the production of active components.

The results generated can be extrapolated to other fermentation products comprising the compound of formula (I) and, optionally, the compound of formula (II).

In a first aspect, the invention provides a fermentation product of a fermenting micro-organism comprising a compound of formula (I): 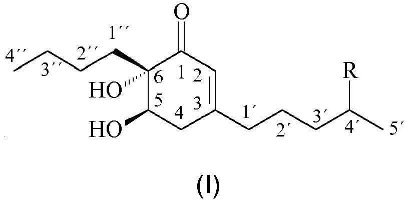 where R is selected from H and OH,
or an isomer, or a salt or a solvate thereof.

In one embodiment of the invention, optionally in combination with any of the subsequent embodiments, "fermentation product" means a fermentation product which is the result of the action of a fermenting micro-organism which, when grown in a suitable culture medium, has the ability to synthesize at least one compound of formula (I). The term "fermentation product" includes the product obtained directly from the fermentation process as well as those obtained after subjecting the product resulting from the fermentation process to subsequent steps of extraction, purification and/or concentration. Thus, the fermentation product of the invention may be in crystalline form as free compounds or as solvates. Solvation methods are generally known in the state of the art.

In the present invention "fermenting micro-organism" means any micro-organism or combination of micro-organisms, with the ability to ferment and to produce, during that process, a compound of formula (I).

In another embodiment, optionally in combination with any of the subsequent embodiments, "fermentation product" means a fermentation product which is the result of the action of an endophytic fungus of the species *Stemphylum solani* which, when grown in a suitable culture medium, has the ability to synthesize at least one compound of formula (I). Preferably the endophytic fungus is the Aa22 strain of *S*. *solani* with deposit number CECT 20941. The fermentation product may be a dry extract obtained by extraction with pre-filtration with an organic solvent, or a freeze-dried product.

In one embodiment of the first aspect, the fermentation product comprises a compound of formula (Ia), or an isomer, salt or solvate of (Ia), and/or a compound of formula (Ib), or an isomer, salt or solvate of (Ib):

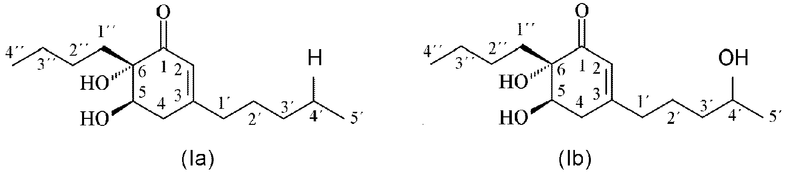

In one embodiment of the first aspect, optionally in combination with any of the embodiments provided above or below, the fermentation product further comprises a compound of formula (II): 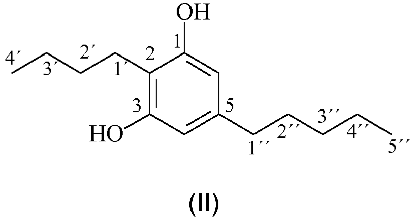 or an isomer, or a salt or a solvate thereof.

Generally, all compounds included in the scope of the invention may include isomers, depending on the presence of multiple bonds, including optical isomers or enantiomers, depending on the presence of chiral centres. The isomers, enantiomers or individual diastereoisomers and mixtures thereof fall within the scope of the present invention, i.e., the term isomer also refers to any mixture of isomers, such as diastereomers, racemates, etc., including their optically active isomers or mixtures in different proportions thereof. The individual diastereoisomers or enantiomers, and mixtures thereof, can be separated by conventional techniques.

Also, within the scope of this invention, are salts and acceptable solvates of all the compounds included within the scope of the invention or of any other compound which, when applied to an organism harmful to plants, is capable of providing (directly or indirectly) a compound as described herein.

In another embodiment of the first aspect, optionally in combination with any of the embodiments provided above or below, the fermentation product is a fermentation extract.

In the present invention the term fermentation "extract" is used with its conventional meaning to refer to preparations of liquid, semi-solid or solid consistency, which are concentrated or unconcentrated, obtained by subjecting the product obtained from the fermentation process to one or more extraction steps. Appropriate means to perform the extraction steps include, for example, the use of organic solvents, microwaves or supercritical fluid extraction.

In another embodiment of the first aspect, optionally in combination with any of the embodiments provided above or below, the fermentation product is free of the fermenting micro-organism.

In the present invention the term "fermentation product free from fermenting micro-organism" means the product resulting from subjecting the product from the fermentation and/or purification or concentration step, to a step of removing the micro-organism, so that the resulting fermentation product lacks viable cells, mycelia or endospores.

In another embodiment of the first aspect, optionally in combination with any of the embodiments provided above or below, the fermentation product comprises the fermenting micro-organism inactivated.

In the present invention, the term "fermentation product comprising the fermenting organism inactivated" refers to a fermentation product according to the first aspect of the invention in which the micro-organism has been inactivated in a step subsequent to the fermentation step. The term "inactivated" means that the micro-organism is not able to form colonies. In one embodiment, the inactivated micro-organisms have the cell membrane intact or broken.

In another embodiment of the first aspect of the invention, optionally in combination with any of the embodiments provided above or below, the fermenting micro-organism is a fungus.

In another embodiment of the first aspect of the invention, optionally in combination with any of the embodiments provided above or below, the fermenting micro-organism is an endophytic fungus.

In another embodiment of the first aspect of the invention, optionally in combination with any of the embodiments provided above or below, the fermenting micro-organism is an endophytic fungus of the genus *Stemphylium.*

In another embodiment of the first aspect of the invention, optionally in combination with any of the embodiments provided above or below, the fermenting micro-organism is an endophytic fungus of the species *Stemphylium solani.*

In another embodiment of the first aspect of the invention, optionally in combination with any of the embodiments provided above or below, the fermenting micro-organism is the strain of *Stemphylium solani* with deposit number CECT 20941.

In a second aspect, the invention relates to a compound of formula (I): 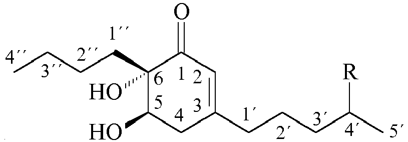 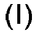 where R is selected from H and OH,
or an isomer, salt or solvate thereof, which has broad-spectrum biocidal activity, hereinafter compound of the invention, and which is comprised in the fermentation product of the invention, either alone or combined with at least the compound of formula (II) as defined herein.

While the compound of formula (I) has been isolated from a fermentation product, a person skilled in the art, using his general knowledge, may prepare said compound by other means.

In the scope of the present invention, the terms "compound of formula (I) wherein R=H", "*Stempholone A*"*,* and compound of formula (Ia), are used interchangeably; just as "compound of formula (I) where R=OH", "*Stempholone B*" and compound of formula (Ib), are used interchangeably; just as "compound of formula (II)" and "*Stemphol*" are used interchangeably.

In a third aspect, the invention provides a process for preparing the fermentation product defined in the first aspect of the invention, comprising (a) a fermentation step comprising culturing the fermenting micro-organism in a suitable culture medium and under suitable conditions; and optionally (b) one or more extraction steps.

In a particular embodiment, the process of the invention allows the fermentation product of the invention to comprise a compound of formula (I) wherein R is H and/or another compound of formula (I) wherein R is OH, or an isomer, or a salt or a solvate thereof.

In another more particular embodiment than the above, the process of the invention further achieves that, the fermentation product of the invention comprises the compound of formula (II).

While there are useful commercial culture media in fermentation processes, the person skilled in the art is able to design the most suitable culture medium for the particular fermenting micro-organism having the ability to produce the compound of formula (Ia), of formula (Ib), or the ability to produce both (Ia) and (Ib), or the ability to produce (Ia), (Ib) or (Ia) and (Ib) together with the compound of formula (II).

Thus, for fermentation to take place, the micro-organism requires a carbon source from which to extract the energy necessary for its metabolism. The most common carbon sources are carbohydrates such as starch and sugars. In the search for new carbon sources, most recently, the use of lignocellulosic resources (cereal straw, trees and waste, etc.) as the main source of renewable biomass, is being studied.

Many of these carbon sources require pretreatment prior to their use; as is the case, for example, of starch, which must be cooked and hydrolyzed to glucose before being transformed into ethanol by micro-organisms that perform this transformation. It is also the case of cellulose and the lignocellulosic substrates in general, which need drastic physical and/or chemical treatments before being used for this purpose.

Other nutrients that are needed in large quantities for microbial growth are nitrogen, phosphorus and sulphur. These elements are incorporated into the cell's structural and functional molecules. Nitrogen, in particular, must be provided in varying proportions in the form of protein nitrogen obtained from by-products of the corn industry, yeast extract or others, and non-protein nitrogen (ammonium salts, urea, etc.). The other two elements are delivered as phosphate and sulphate salts, respectively.

Finally, a number of micro-nutrients (vitamins, iron, cobalt, copper, zinc, etc.), must be supplied to the medium.

Fermentation can be of various types, including liquid fermentations, solid state fermentation (SSF), or submerged solid fermentation (SmF).

As for the conditions of the fermentation reaction, the person skilled in the art is able to adjust the main variables, which are temperature, acidity and oxygen pressure, which are determined by the nature of the micro-organism itself.

In one embodiment of the process of the third aspect of the invention, the fermentation step is carried out in a culture medium comprising the necessary nutrients for fungal growth (including sources of carbon, nitrogen, phosphorus, sulphur and micronutrients (vitamins, iron, cobalt, copper, zinc, etc.)).

In one embodiment of the process of the third aspect of the invention, when the fermenting micro-organism is a fungus, the fermentation step is carried out in a culture medium comprising the necessary nutrients for fungal growth (including sources of carbon, nitrogen, phosphorus, sulphur and micronutrients (vitamins, iron, cobalt, copper, zinc, etc.)).

In one embodiment of the process of the third aspect of the invention, when the fermenting micro-organism is a fungus, the fermentation step is carried out in the presence of a solid vegetable product. In the present invention, "solid vegetable product" referred to in this embodiment of the third aspect of the invention means a part of a plant (fruit, seed (including cereal grains such as rice, corn, wheat, etc.), leaves, stems, roots, tubers, derivative products (flour, bran, pulp) as well as by-products (bagasse, straw, husks, shells, pulp, biomass, liquor, mucilage, olive pomace paste, fibre, lignocellulosic waste). In one embodiment of the process of the third aspect of the invention, when the fermenting micro-organism is a fungus, the fermentation step is carried out in the presence of a solid vegetable product, which is a cereal that may be whole (i.e. as is when isolated from nature) or processed (for example, ground). Illustrative and non-limiting examples of cereals include rice. The amount and treatment to which the cereal is subjected prior to its use in fermentation, are routine acts for the person skilled in the art.

In one embodiment of the process of the third aspect, optionally in combination with any of the embodiments provided above or below, the fermentation step is carried out in the dark.

In one embodiment of the process of the third aspect, optionally in combination with any of the embodiments provided above or below, the fermentation step is carried out at room temperature (i.e. at a temperature between 20-27 °C). In one embodiment of the process of the third aspect, optionally in combination with any of the embodiments provided above or below, the fermentation step is carried out at room temperature (i.e. at a temperature between 24-27 °C).

In one embodiment of the process of the third aspect, optionally in combination with any of the embodiments provided above or below, the step (b) of extraction of the fermentation product is carried out by extraction with suitable organic solvents.

There are three types of solvent, depending on their polarity: (a) protics (e.g., water, carboxylic acids, alcohols, amines), characterised in that they have a functional group capable of providing protons (e.g., OH, NH, SH), as well as the capacity to form hydrogen bridges; (b) polar aprotics (e.g., DMSO, DMF, HMPA, nitriles, ketones, nitro-compounds) characterised in that they lack functional groups able to transfer protons and that they have a high dielectric constant; and (c) apolar aprotics (e.g., (aliphatic, aromatic, halogenated), ethers, esters, alkyl halides) characterised in that they lack functional groups capable of providing protons and a low dielectric constant.

In one embodiment of the process of the third aspect, optionally in combination with any of the embodiments provided above or below, the solvent or mixture of solvents used to carry out the extraction are of the polar aprotic type. In another embodiment of the process of the third aspect, optionally in combination with any of the embodiments provided above or below, the extraction of the fermentation product is carried out with an organic solvent which is ethyl acetate.

In an embodiment of the process of the third aspect, optionally in combination with any of the embodiments provided above or below, the process includes a step prior to step (a), wherein the fermenting micro-organism is let grow in a suitable culture medium until an optimal amount of mycelia is formed for starting step (a).

In one embodiment of the process of the third aspect, optionally in combination with any of the embodiments provided above or below, the process additionally comprises a step (i.1), wherein the fermenting micro-organism is removed.

The removal of the micro-organism from the fermentation product may be carried out using well-established techniques such as ultra-centrifugation or filtration.

In one embodiment of the process of the third aspect, optionally in combination with any of the embodiments provided above or below, the process additionally comprises a step (i.2), wherein the fermenting micro-organism is inactivated.

The inactivation of the micro-organism may be carried out using techniques well known by the person skilled in the art, such as cell lysis.

In another embodiment of the process of the third aspect, optionally in combination with any of the embodiments provided above or below, the process comprises a subsequent step (d) of purification of the fermentation product resulting from step (b), (i.1.) or (i.2.). In another embodiment of the process of the third aspect, optionally in combination with any of the embodiments provided above or below, the purification step (d) is carried out by means of chromatographic techniques wherein the eluate corresponds to the purified fraction of the fermentation product of the invention. The eluent to carry out the chromatographic separation may be organic solvents of increased polarity (e.g., hexane, dichloromethane, ethyl acetate, acetone, methanol, etc.) or combinations thereof.

In another embodiment of the process of the third aspect, optionally in combination with any of the embodiments provided above or below, the process additionally comprises a drying step of the product resulting from step (b), (i.1.), (i.2.) and/or (d).

In another embodiment of the process of the third aspect, optionally in combination with any of the examples provided above or below, the step of drying the fermentation product comprises the extraction with organic solvent and its concentration (prior filtration of the mycelia), or, alternatively, by vacuum drying (for example by using a rotary evaporator).

Examples of organic solvents, which are used in the field of the invention, are dichloromethane, ethyl ether and preferably ethyl acetate.

In another embodiment of the third aspect, optionally in combination with any of the embodiments provided above or below, the fermenting micro-organism is a fungus. In another embodiment of the third aspect, optionally in combination with any of the embodiments provided above or below, the fermenting micro-organism is an endophytic fungus.

The type of fungus and its interaction with the host plant confers certain characteristics that enable the more or less favourable production of certain compounds.

In another embodiment of the third aspect of the invention, the micro-organism is a species of *Stemphylium solani,* which due to its interaction with the host plant, is efficient in the production of the compounds of formula (I) and (II). Preferably, the fermenting micro-organism is the Aa22 strain of *S*. *solani* isolated from leaves of *Artemisia absinthium* deposited on the date of 25 of September 2015 in the Spanish Type Culture Collection with number CECT 20941, following the Budapest Treaty on the International Micro-organism Deposit System for the purposes of patent procedure.

Strains of *S*. *solani* can be isolated from other plants, such as, for example, *Arabidopsis thaliana.*

Examples of culture media that allow the fermentation by fungi of the species *Stemphylium solani* are YMB (yeast extract and malt extract broth), the media cited in the references such as Molitor et al. 2012. J. Nat. Prod. 75, 1265-1269) and Buckel et al. 2013. Phytochemistry 89, 96-103); those which are cited in the document WO2002017937 A1 or any other commercial culture medium for phytopathogenic fungi.

In a particular embodiment, the endophytic fungus is the Aa22 strain of *Stemphylium solani* isolated from plant material of *Artemisia absinthium* deposited in the Spanish Type Culture Collection with number CECT 20941 on 25 September 2015, which is cultivated using a YMB.

In a particular embodiment, the endophytic fungus is the Aa22 strain of *Stemphylium solani* isolated from plant material of *Artemisia absinthium* deposited in the Spanish Type Culture Collection with number CECT 20941 on 25 September 2015, which is cultivated using a YMB in a step prior to fermentation.

In another particular embodiment of the third aspect of the invention, optionally in combination with any of the embodiments provided above or below, the endophytic fungus is the Aa22 strain of *Stemphylium solani* isolated from plant material of *Artemisia absinthium* and deposited in the Spanish Type Culture Collection with number CECT 20941 on 25 September 2015, which (1) is grown in a YMB culture medium, (2) subsequently, it undergoes fermentation in the presence of rice, and (3) the resulting product from the fermentation is subjected to extraction with a suitable organic solvent. Step (1) is carried out until bringing the fungus to the optimal stage for the inoculation of step (2) and the production, in the desired amount in step (2), of the compound of formula (I) and, optionally, of the compound of formula (II). Step (3) may be carried out in the presence of a solvent or mixture of polar aprotic solvents. In another embodiment, step (3) is carried out in the presence of ethyl acetate.

In another particular embodiment of the third aspect of the invention, optionally in combination with any of the embodiments provided above or below, the endophytic fungus is the Aa22 strain of *Stemphylium solani* isolated from plant material of *Artemisia absinthium* and deposited in the Spanish Type Culture Collection with number CECT 20941 on 25 September 2015 (1), which is grown in YMB, subsequently, (2) it is subjected to fermentation in the presence of rice, and (3) the resulting product from the fermentation is subjected to 1, 2, or 3 steps of extraction with a suitable organic solvent. If two or three extraction steps are carried out, each one is carried out using the same solvent or different solvents. In another embodiment of step (3), each extraction step is carried out with a solvent or mixture of polar aprotic solvents. In another embodiment of step (3), each extraction step is carried out in the presence of ethyl acetate.

In another particular embodiment of the third aspect of the invention, optionally in combination with any of the embodiments provided above or below, the process is one wherein the endophytic fungus is the Aa22 strain of *Stemphylium solani* isolated from plant material of *Artemisia absinthium* deposited in the Spanish Type Culture Collection with number CECT 20941 on 25 September 2015 and the cultivation of the fungus is carried out in a YMB medium, the subsequent fermentation in the presence of rice, and the extraction of the resulting product from the fermentation comprises 3 extraction steps with a suitable organic solvent. In another particular embodiment of the third aspect of the invention, optionally in combination with any of the embodiments provided above or below, the process is one wherein the endophytic fungus is the Aa22 strain of *Stemphylium solani* isolated from plant material of *Artemisia absinthium* deposited in the Spanish Type Culture Collection with number CECT 20941 on 25 September 2015 and the cultivation of the fungus is carried out in a YMB medium, the subsequent fermentation in the presence of rice, and the extraction of the resulting product from the fermentation comprises 3 extraction steps with a suitable polar aprotic solvent. In another particular embodiment of the third aspect of the invention, optionally in combination with any of the embodiments provided above or below, the process is one wherein the endophytic fungus is the Aa22 strain of *Stemphylum solani* isolated from plant material of *Artemisia absinthium* deposited in the Spanish Type Culture Collection with number CECT 20941 on 25 September 2015 and the cultivation of the fungus is carried out in a YMB medium, the subsequent fermentation in the presence of rice, and the extraction of the resulting product from the fermentation comprises 3 extraction steps with ethyl acetate.

In a fourth aspect, the present invention provides a fermentation product comprising the compound of formula (I) as defined in the second aspect of the invention, obtainable by the process as defined in the third aspect of the invention.

In one embodiment of the fourth aspect of the invention, the fermentation product further comprises the compound of formula (II) as defined above.

In a fifth aspect, the present invention provides a process for preparing a compound of formula (I) as defined in the second aspect of the invention or of the compound of formula (II) as defined above, comprising the step of isolating the compound of formula (I) or of the compound of formula (II) from the product resulting from step (b), (i.1.), (i.2) or (c) defined above.

The isolation of the compounds (I) and (II) can be carried out by fractionation techniques, including chromatography.

Fractionation techniques known to any person skilled in the art, and that allow obtaining the compounds within the scope of the invention are, for example, and without limitation, vacuum liquid chromatography (VLC), column chromatography (CC), column chromatography (CC) using different solid phases (Silica Gel, Sephadex LH-20) and high performance liquid chromatography (HPLC), being eluted with gradients of different polarity through different combinations of organic solvents.

In one embodiment of the fifth aspect of the invention, the isolation of the compounds (I) and (II) is carried out by chromatography. In another embodiment of the fifth aspect of the invention, the isolation of the compounds (I) and (II) is carried out by silica gel column chromatography, using mixtures of solvents with increasing polarity. In another embodiment of the fifth aspect of the invention, the isolation of the compounds (I) and (II) is carried out by column chromatographic separation in silica gel of the resulting product of the step (b), (i.1.) or (i.2.), using mixtures of solvents of n-hexane/ethyl acetate and acetone with increasing polarity. In one embodiment of the fifth aspect, the chromatographic separation is carried out in silica gel column using n-hexane/ethyl acetate gradients with increasing polarity from 90:10 (v/v) to 50:50 (v/v). In another embodiment of the fifth aspect, the chromatography in silica gel is carried out using n-hexane/ethyl acetate with increasing gradients in polarity from 90:10 (v/v) to 60:40 (v/v). In another embodiment of the fifth aspect of the invention, subsequent to the elution with hexane/ethyl acetate, the extract loaded in the column is subjected to an elution with 100% acetone.

In the present invention, "v/v" means the volume ratio of the solvents constituting the mobile phase of the chromatography necessary to separate the fractions with the compounds of formula (I) and/or (II).

In the present invention the "silica gel column chromatographic separation using mixtures of n-hexane/ethyl acetate, acetone and/or methanol/water with increasing polarities" comprises carrying out the separation in a silica gel chromatographic column using n-hexane/ethyl acetate, acetone or methanol/water, or by carrying out the elution in various stages, combining the eluents. The person skilled in the art, using his knowledge, can optimise ratios between different solvents and can set the order of the eluents depending on the nature of the molecule to be eluted.

In one embodiment of the fifth aspect, the process is for preparing a compound of formula (Ia) and comprises (i) subjecting the resulting fermentation product of step (b) to silica gel column chromatographic separation using mixtures of n-hexane/ethyl acetate with increasing polarities and, subsequently, acetone (ii) collecting the fraction eluted with acetone, (iii) subjecting the resulting eluate from step (ii) to silica gel chromatography with mixtures of n-hexane/ethyl acetate with increasing polarities; and (iv) collecting the lower polar fractions. In an embodiment of the fifth aspect, the step (i) comprises carrying out a silica gel chromatography, which is carried out using n-hexane/ethyl acetate gradients with increasing polarities, from 90:10 (v/v) to 60:40 (v/v). In another embodiment of the fifth aspect of the invention, step (i) comprises, subsequent to the elution with hexane/ethyl acetate, elution with 100% acetone. In another embodiment of the fifth aspect, step (iii) comprises subjecting the fraction eluted with acetone to separation by silica gel chromatography using n-hexane/ethyl acetate gradients with increasing polarities, from 80:20 (v/v) to 10:90 % (v/v), and, optionally, subjecting it to a drying step.

In another embodiment of the fifth aspect, the process is for preparing a compound of formula (Ib) and comprises the step (v) subjecting the resulting fermentation product of step (b) to silica gel column chromatographic separation using mixtures of n-hexane/ethyl acetate with increasing polarities and, subsequently, acetone (vi) collecting the fraction eluted with acetone, and (vii) subjecting the resulting eluate from step (vi) to silica gel chromatography with mixtures of n-hexane/ethyl acetate with increasing polarities; and (viii) collecting the higher polar fractions and subjecting them to molecular exclusion chromatography (Sephadex LH-20) with n-hexane/dichloromethane/methanol (at a ratio of, for example 2:1:1 (v:v:v)). In an embodiment of the fifth aspect, the step (v) comprises carrying out a silica gel chromatography, which is carried out using n-hexane/ethyl acetate gradients with increasing polarities, from 90:10 (v/v) to 60:40 (v/v). In another embodiment of the fifth aspect of the invention, step (v) comprises, subsequent to the elution with hexane/ethyl acetate, elution with 100% acetone. In another embodiment of the fifth aspect, step (iii) comprises subjecting the fraction eluted with acetone to separation by silica gel chromatography using n-hexane/ethyl acetate gradients with increasing polarities, from 80:20 (v/v) to 10:90 % (v/v). In another embodiment of the fifth aspect, step (iii) comprises subjecting the fraction eluted with acetone to separation by silica gel chromatography using increasing gradients in polarity of n-hexane/ethyl acetate, from 50:50 (v/v) to 20:80 (v/v), and, optionally, subjecting it to a drying step.

In another embodiment of the fifth aspect, the process is for preparing a compound of formula (II) comprising the step (ix) subjecting the resulting fermentation product of step (b) to silica gel column chromatography using mixtures of n-hexane/ethyl acetate with increasing polarities, from 90:10 (v/v) to 60:40; (x) subjecting the eluate resulting from step (ix) to silica gel column chromatography with mixtures of n-hexane/acetone with increasing polarities, from 90:10 (v/v) to 100 % of acetone; (xi) collecting the highest polar fraction (corresponding to the fractions eluted at 100 % acetone) and, optionally, drying.

In another embodiment of the fifth aspect, the process is for preparing a compound of formula (II) comprising the step (ix) subjecting the resulting fermentation product of step (b) to silica gel column chromatography using mixtures n-hexane/ethyl acetate with increasing polarities, from 90:10 (v/v) to 60:40; (x) subjecting the eluate resulting from step (ix) to silica gel column chromatography with mixtures of n-hexane/acetone with increasing polarities, from 98:2 (v/v) to 90:10 of acetone; (xi) collecting the fractions eluted at 94:6 to 92:8 (v/v) of n-hexane/acetone and subjecting them to silica gel column chromatography with mixtures of n-hexane/dichloromethane with increasing polarities, from 50:50 (v/v) to 100 % dichloromethane, and (xii) collecting the fractions with lower polarity (50:50); and, optionally, subjecting them to a drying step.

In another embodiment of the fifth aspect, the process is for preparing a compound of formula (II) comprising the step (ix) subjecting the resulting fermentation product of step (b) to silica gel column chromatography using mixtures of n-hexane/ethyl acetate with increasing polarities, from 90:10 (v/v) to 60:40; (xiii) subjecting the eluate resulting from the elution with n-hexane/ethyl acetate 80:20 (v/v) to silica gel column chromatography with a mixture of n-hexane/dichloromethane/methanol (50:25:25 (v/v)); (xiv) collecting the eluate; and, optionally, subjecting it to a drying step.

In another embodiment of the fifth aspect of the invention, the process comprises an additional step of purification, concentration and/or drying of the fractions collected in step (iv), (viii), (xi), (xii) and (xiv). Purification, concentration and drying techniques are well known to the person skilled in the art and also have been referred to above herein.

In a sixth aspect, the present invention provides an isolated strain of the endophytic fungus of the species *Stemphylium solani* deposited in the Spanish Type Culture Collection with deposit number CECT20941 or a mutant thereof that maintains the ability to produce the compound of formula (I).

The term "mutant" means a fungus that is derived from the strain CECT20941 of the invention and which is characterised in that it maintains the ability of producing the compound of formula (I) of the invention and, optionally, the compound of formula (II). A mutant of CECT20941 from *Stemphylium solani* is understood as a "variant" CECT20941 of *Stemphylium solani.* The person skilled in the art will understand that the mutants retaining the characteristics and advantages of the strain of the invention can be obtained routinely, for example by spontaneous mutagenesis or directed mutation using the strain of the invention as starting material.

In a seventh aspect, the present invention provides the use of a fermentation product as defined in the first aspect of the invention, or of the fermentation product as defined in the fourth aspect of the invention, or of the compound of general formula (I) as defined in the second aspect of the invention, or the compound of formula (II) as defined below, or of the strain of *Stemphylium solani* of the sixth aspect of the invention as a biocidal agent.

As indicated above, the present invention provides compounds and fermentation products (and consequently the deposited strain that also produces them) leading to a broad-spectrum biocidal activity.

"Broad-spectrum biocidal activity" means the ability to simultaneously control more than one different category of organisms harmful to plants. Such control comprises the prevention of action or the direct destruction of such organisms harmful to public health and also to agriculture during production, but it also extends to storage, transport, distribution and processing of agricultural products and their derivatives.

Examples of categories of organisms harmful to plants, include but are not limited to, insect-pests, fungi or nematodes.

Preferably, the insect-pests that are included in the scope of this invention are herbivorous insect-pests with different trophic adaptations, either chewing or sucking (aphids), and which can have a high impact on horticultural crops causing serious economic losses, developing resistance to synthetic insecticides and presenting virus transmission capacity. Illustrative and non limiting examples of herbivorous insect-pests of different trophic adaptations are *Spodoptera littoralis, Myzus persicae* and *Rhopalosiphum padi* Sp.

The activity against these herbivorous insect-pests can be determined by different types of bioassays including anti-feedant (inhibition of feeding and/or settlement in the case of aphids), repellent or toxic activity, among others.

Preferably, the fungi belong to a species of phytopathogenic fungi. Illustrative and non limiting examples of fungi against which the fermented product of the invention is effective are *Fusarium oxysporum, Fusarium moniliforme, Fusarium solani* and *Botrytis cinerea.*

Antifungal activity can be determined, for example, by inhibition assays of mycelial growth on plate.

Preferably, the nematodes that are included in the scope of the invention are nodule forming nematodes of the roots (*Meloidogyne* sp). An example of nodule forming nematodes is the polyphagous species *Meloidogyne javanica,* with capacity to parasitize more than 3,000 species of crop plants, including extensive crops, horticultural and fruit, seriously affecting production (Agrios 2005. Plant Pathology, Fifth edition, Elsevier/Academic, Amsterdam), and causing annual economic losses of billions of euros (Singh et al. 2013. OEPP/EPPO Bulletin 43 (2), 334-374).

The activity against nematodes is related to toxicity, i.e. the ability to disrupt a particular phase of the life cycle of the nematode preventing its development. In the scope of the invention, the nematicidal activity can be determined, for example, by determining the percentage of infective juveniles (J2) dead after 72 hours subsequent to the application of the fermentation product, compound of formula (I), strain or biocidal composition of the invention.

In a ninth aspect, the invention provides a biocidal composition comprising the fermentation product as defined in the first aspect of the invention, or the fermentation product as defined in the fourth aspect of the invention, or the compound of formula (I) as defined in the second aspect of the invention, or the compound of formula (II) as defined below, or the strain of *Stemphylium solani* of the sixth aspect of the invention.

The biocidal composition of the invention may further comprise various carriers and agents to facilitate its conservation, handling and application.

As the person skilled in the art will know, in the application of phytosanitaries, normally solid carriers, liquid carriers, gaseous carriers, etc., are used, and, if necessary, surfactants and auxiliary agents for the formulation of phytosanitary compositions such as, for example, an additive to formulate forms such as emulsifiable concentrates, wettable powders, flowable liquids (e.g., water suspension, water emulsion, etc.), powders, aerosols, ULV and the like.

Examples of solid carrier included in the scope of the invention are fine powders or clay granules (e.g. kaolin clay, diatomaceous earth, synthetic hydrated silicon oxide, bentonite, Fubasami clay, acid clay, etc.), talcs, ceramics and other inorganic minerals (e.g., sericite, quartz, sulphur, active carbon, calcium carbonate, hydrated silica, etc.), commercial fertilizers (e.g., ammonium sulphate, ammonium phosphate, ammonium nitrate, urea, ammonium chloride, etc.) and the like.

Examples of liquid carrier that are included in the scope of the invention are water, alcohols (e.g., methanol, ethanol, etc.), ketones (e.g., acetone, methyl ethyl ketone, etc.), aromatic hydrocarbons (e.g., benzene, toluene, xylene, ethylbenzene, methylnaphthalene, etc.), aliphatic hydrocarbons (e.g., hexane, cyclohexane, kerosine, gas oil, etc.), esters (e.g., ethyl acetate, butyl acetate, etc.), nitriles (e.g., acetonitrile, isobutyronitrile, etc.), ethers (e.g., diisopropyl ether, dioxane etc.), acid amides (e.g., N, N-dimethylformamide, N, N-dimethylacetamide, etc.), halogenated hydrocarbons (e.g., dichloroethane, trichloroethane, tetrachloride carbon, etc.), dimethylsulfoxide, vegetable oils (e.g., soybean oil, cottonseed oil, etc.) and the like.

Examples of gas carriers which are included in the scope of the invention are spray agents including flon gas, butane gas, LPG (liquefied petroleum gas), dimethyl ether, carbon dioxide gas and the like.

Examples of surfactants included in the scope of the invention are alkyl sulphates, salts of alkyl sulphonate, alkyl aryl sulphonates, alkyl aryl esters, polyoxyethylene compounds thereof, polyethylene glycol esters, polyhydroxy alcohol esters, sugar alcohol derivatives, and the like.

Examples of auxiliary agents for formulation such as fixing agent and dispersing agent, included in the invention, are casein, gelatin, polysaccharides (e.g., starch powder, arabic gum, cellulose derivative, alginic acid, etc.), lignin, bentonite and sugar derivatives, synthetic water-soluble polymers (e.g., polyvinyl alcohol, vinyl polypyrrolidone, acrylic polyacids, etc.), and the like.

Examples of stabilisers which are included in the scope of the invention are PAP (isopropyl acid phosphate), BHT (2,6-di-tert-butyl-4-methylphenol), BHA (mixture of 2-tert-butyl-4-methoxyphenol and 3-tert-butyl-4-methoxyphenol), vegetable oils, mineral oils, surfactants, fatty acids or esters thereof and the like.

The fermentation product of the invention, the compound of the invention, the compound of general formula (II) and the biocidal composition of the invention can be used together with at least one additional active ingredient. Examples of additional active ingredients are nematicides, insecticides, acaricides, fungicides, herbicides, plant growth regulators, synergists, fertilizers, soil conditioners and animal baits.

In a tenth aspect, the present invention provides the use of the fermentation product of the invention, or of the compound of the invention, or of the compound of formula (II), either alone or in combination, to prepare a biocidal composition.

In an eleventh aspect, the invention relates to the use as a broad-spectrum biocidal agent for the control of harmful organisms, affecting plants, hereinafter use of the invention, of the fermentation product of the invention, or of the compound of the invention, of a compound of formula (II), of the strain of the invention or of the biocidal composition of the invention, either alone or in combination. Preferably the use of the invention is effective simultaneously against more than one category of harmful organisms, which are selected from insect-pests, fungi and at least one nematode.

In a particular embodiment, the use of the invention as a broad-spectrum biocidal agent comprises at least the fermentation product of the invention, the compound (I) of the invention, a compound of formula (II), or the strain of the invention and it is simultaneously active against insect-pests and fungi.

In a particular embodiment, the use of the invention, as a broad-spectrum biocidal agent comprises at least the fermentation product of the invention, the compound (I) of the invention, a compound of formula (II), or the strain of the invention and it is simultaneously active against, at least, insect-pests, fungi and, at least, one nematode.

In another particular embodiment, the use of the invention comprises at least the fermentation product as defined in the first and fourth aspects, and it is simultaneously active against insect-pests, fungi and, at least, one nematode. In another particular embodiment, the use of the invention comprises at least one compound of formula (I) and it is simultaneously active against insect-pests, fungi and, at least, one nematode. In another particular embodiment, the use of the invention comprises, at least, one compound of formula (Ia) and it is simultaneously active against insect-pests, fungi and, at least, one nematode.

In another particular embodiment, the use of the invention comprises the compound of formula (II) and it is simultaneously active against insect-pests and fungi.

In a final aspect, the invention relates to a method of broad-spectrum control of harmful organisms affecting plants, hereinafter control method of the invention, comprising administering an effective dose of the biocidal composition of the invention, or of the fermentation product of the invention, or of the compound of the invention, of a compound of formula (II), or of the strain of the invention, either alone or in combination, to the plant or to the substrate (understood as the material that serves as a "settle" for a plant). Preferably, the control method of the invention is effective simultaneously against more than one category of harmful organisms, which are selected from insect-pests, fungi and, at least, one nematode.

In a particular embodiment, the control method of the invention is simultaneously active against insect-pests and fungi.

In a particular embodiment, the control method of the invention is simultaneously active against, at least, insect-pests, fungi and, at least, one nematode.

Application may be directly by spraying where the harmful organism is located (either on the plant, or on the substrate).

The "effective dose" is the amount that proves efficient in the control of harmful organisms. The person skilled in the art can routinely determine the effective dose. Factors that determine the required amount to be applied are: (a) if the biocidal composition of the invention, or the fermentation product of the invention, or the compound (I) of the invention, the strain or a compound of formula (II) is used either alone or in combination, (b) the type of formulation, (c) the moment, (d) where and how it is applied, (e) the type of harmful organism affecting the plant and (f) the degree of damage.

In one embodiment, the control method is a preventive method, i.e., the fermentation product, compound (I), compound (II), strain or biocidal composition is applied on the substrate or on those parts of the plant that are colonised by the harmful organisms, said application being carried out before the harmful organisms start damaging the plant.

Alternatively, in another embodiment, the control method is a method of treatment, i.e., the fermentation product, compound (I), compound (II) strain or biocidal composition is applied in those areas of the plant already damaged by colonisation by harmful organisms.

Throughout the description and claims the word "comprise" and its variations are not intended to exclude other technical characteristics. For the skilled person in the art, other objects, advantages and characteristics of the invention will arise partly from the description and partly from the practice of the invention. The following examples are provided as an illustration, and are not intended to be limiting of the present invention.

### MODE FOR CARRYING OUT THE INVENTION

### A) METHOD TO OBTAIN A FERMENTATION PRODUCT AND CHARACTERISATION OF ITS COMPOUNDS WITH BIOCIDAL ACTIVITY

### Example 1. Isolation and identification of the Aa22 strain of the endophytic fungus S. solani

The Aa22 strain of the endophytic fungus *S*. *solani* was isolated from leaves of *Artemisia absinthium* collected in Terceira (The Azores, Macaronesia region). The fresh plant material was superficially sterilised with sodium hypochlorite (65 %), ethanol (75 %) and sterile distilled water. Small samples of explants (leaves and stems) were incubated at 27 °C in the dark in two culture media (PDA and YMB) in petri plates with 50 mg/l of antibiotic to prevent contamination. The Aa22 pure strain of *S*. *solani* was obtained by individually isolating a colony grown in YMB medium and it was replicated in the same conditions for its maintenance.

The morphological identification of the strain of the fungus was performed on a microscope according to the characteristics of the mycelia, spores and reproductive structures, by dying small samples of the isolated colonies with methylene blue. Likewise, its identification was carried out at molecular level by (PCR) amplification and sequencing of the ribosomal ITS region of the rDNA extracted from a sample of the mycelia (Arenal et al. 2000. Mycological Research 104, 2000, 301-303; Giménez. 2006. Bioactive products of Canary Island plants and endophytes: activity detection and use in controlling agricultural pests and diseases. PhD thesis, University of La Laguna). The ITS1-5.8S-ITS2 sequence of the rDNA was compared with those published in the NCBI databases (*National Centre for Biotechnology Information*) (access number GenBank JF913269.1). Having identified the Aa22 strain of the endophytic fungus *S*. *solani,* it was deposited in the Spanish Type Culture Collection (CECT) on 25 September 2015 with corresponding access number CECT 20941, following the Budapest Treaty on the International Microorganism Deposit System for the purposes of patent procedure.

### Example 2. Method to obtain the fermentation product of the Aa22 strain of S. solani

From a two week culture in YMB medium (yeast extract and malt extract broth to which agar is added) in petri plate of the Aa22 strain of *S*. *solani,* six samples of fragments (2.5x2.5 cm) of fresh mycelia were taken and inoculated in twelve sterile Erlenmeyer flasks (6 fragments in each flask) with 100 g of rice and 30 ml of distilled water (to prevent dehydration of the rice). After three weeks of incubation in the dark at 25 °C, the culture was extracted three times with ethyl acetate. To this end, ethyl acetate was added until completely covering the culture medium (120 ml) and was allowed to soak for 48 hours. Subsequently, the suspension was filtered through a funnel with filter paper collecting the content in a container. This extraction process with ethyl acetate was performed three times.

The filtrate was brought to dryness (to evaporate the ethyl acetate) in vacuum in a rotary evaporator to obtain the crude extract (4.58 g).

### Example 3. Isolation and characterisation of the compounds with biocidal activity from the crude extract

### Experimental techniques

The NMR Spectra of ¹H and ¹³C of the compounds identified in the different fractions of the crude extract were recorded in spectrometers Bruker Advance and AMX-500, at 400 and 500 MHz for ¹H and 100 to 125 MHz for ¹³C, respectively. The products were dissolved in deuterochloroform (CDCl₃) containing tetramethylsilane (TMS) as internal standard reference. The multiplicity of the ¹³C signals was determined with broadband decoupling experiments (DEPT, *Delay enhancement of polarisation transfer*). The bidimensional (2D) programs used in the NMR experiments (COSY, NOESY, HSQC and HMBC) were provided by the Bruker company. The high and low resolution mass spectra were recorded in a Micromass Autospec® spectrometer using the technique of electron impact (EI-MS) at 70 eV and a temperature of 220 °C. The preparative and semi-preparative chromatographies were carried out in a liquid flash chromatography instrument (Flash Master Personal, Jones Chromatography) on silica columns (*Isolute flash silica,* 20 g/70 mL, International Sorbent Technology Ltd. Tucson, USA). In the vacuum liquid (VLC) and the column (CC) chromatographies silica gel 0.025-0.04 and 0.040-0.015 mm (Macherey-Nagel GmbH&Co.KG, Düren, Germany) was used as support. In the molecular exclusion chromatographies the support was Sephadex LH-20 (Pharmacia Fine Chemicals). Visualisation of the compounds in thin layer chromatographies (TLC) was performed with a solution of H₂SO₄ (5 %) and vanillin (5 %) in EtOH.

For its part, the crude extract was analysed by LC-MS.

### 3.A. Isolation of compounds with biocidal activity from the crude extract

The methanol-soluble fraction (4.09 g) of the crude extract (4.58 g) obtained according to Example 2, was chromatographed on silica gel (0.3 kg) by vacuum liquid chromatography (VLC) using as eluent mixtures with increasing polarity of n-hexane/ethyl acetate, acetone and methanol/water, yielding 8 fractions: H1-H2-H3-H4-H5-H6 and H8.

H2 fraction (n-hexane/AcOEt, 5:1, 0.575 g) was column-chromatographed (CC) on silica gel using mixtures of n-hexane/acetone (from 2-10 %). Once the highest polar fraction was identified, 42 mg of **stemphol** were obtained by subjecting it to dryness in a rotary evaporator (Stodola et al.1973. Phytochemistry 12, 1797-1798, Marumo et al. 1985. Agric. Biol. Chem. 49, 1521-1522). The other fractions were collected in 6 fractions (H2A-H2F). The fraction H2E (0.053 g) (94:6 to 92:8 (v/v) of n-hexane/acetone) was once again chromatographed on a silica gel column using mixtures of n-hexane/dichloromethane (50:50 (v/v) in 100 % dichloromethane). In the fractions of lower polarity (50 % dichloromethane), after subjecting them to dryness in rotary evaporator, 21 mg of stemphol were obtained.

From the fraction H3 (n-hexane/AcOEt, 4:1, 107 mg), the stemphol was isolated again by chromatography on Sephadex LH-20 with a mixture of n-hexane/dichloromethane/methanol (2:1:1), after subjecting the collected fractions to dryness in the rotary evaporator (74.3 mg).

The fraction H6 (100% acetone, 0.452 g) was chromatographed on a CC of silica gel and eluted with increasing polarity mixtures of n-hexane/ethyl acetate 20-100 % (80:20 (v/v) to 100 % ethyl acetate). In the lower polar fractions (EtOAc 20-30 %) obtained from this fraction 6 the **stempholone A** (19 mg) was isolated by drying in rotary evaporator. From the higher polar fractions (EtOAc 50-80 %), after subjecting them to chromatography on Sephadex LH-20 with n-hexane/dichloromethane/methanol (2:1:1) and drying in the rotary evaporator, 11 mg of the **stempholone B** were obtained,

### 3.B. Characterisation of the compounds with biocidal activity

### Stemphol

The stemphol was isolated from the crude extract as a white solid. The high resolution mass spectrum showed a molecular ion at 236.1772 *m*/*z* (calcd. 236.1776), which was consistent with the molecular formula C₁₅H₂₄O₂ The absorption bands in the IR spectrum at 3300 and 1630 cm⁻¹ indicated the presence of hydroxyl groups and double bonds, respectively. Its ¹H NMR spectrum showed characteristic signals of two alkyl chains (butyl and n-pentyl), the signal of a singlet that integrated two protons at δ 6.22 (H-4, H-6), and two hydoxyl groups at δ 4.62 (2H, s, OH). The chemical shift and multiplicity suggested the presence of a 1,3-dihydroxyphenyl-2,5-tetra-substituted ring. This suggestion was confirmed by the similarity of their spectroscopic data with those published for analogous compounds (Pohanka et al. 2006. J Nat Prod.69, 654-657).

The ¹³C NMR spectrum showed twelve signals corresponding to a methyl group, seven methylene groups, a methine group and three quaternary carbons, which were consistent with the correlations observed in the HSQC experiment. The signals at δ 108.1, 114.1, 142.1, and 154.4 due to aromatic carbons confirmed the presence of the 1,3-dihydroxy-2,5-tetra-substituted phenyl group.

The relative position of substituents was confirmed in the HMBC experiment, with the observed correlations between protons at δ 2.58 (H-1') and δ 6.22 (H-4, H-6) with signals at δ 154.4 (C-1, C-3) and δ 112.5 (C-2), and proton at δ 2.44 (H-1") with δ 108.1 (C-4, C-6) and δ 142.2 (C-5).

These data are consistent with the stemphol, a compound previously isolated from fungi *S*. *majusculum* (Stodola et al.1973. Phytochemistry 12, 1797-1798) and *S*. *botryosum* (Marumo et al. 1985. Agric. Biol. Chem. 49, 1521-1522). Its 2D NMR spectroscopic data allowed assigning all the proton and carbon signals present in the molecule (see Table 1) and identifying its structure as 5-butyl-2-pentyl-resorcine.
Stemphol. IR (film)ₘₐₓ, 3300, 2850 1630 1588, 1430, 1270 cm⁻¹; for ¹H NMR data (CDCl₃, 500 MHz) see Table 1; for ¹³C NMR data (CDCl₃, 125 MHz) see Table 1; HREIMS m/z 236.1771 [M]⁺ (calculated for C₁₅H₂₄O₂, 236.1776); EIMS 70 eV m/z (rel. int.): 236 [M⁺] (16), 194 (16), 193 (100), 180 (45), 137 (7), 123 (11), 91 (3), 77 (4).

**Table 1. Spectroscopic data from ¹H NMR and ¹³C NMR of the stemphol**

| H/C | ¹H NMR | ¹³C NMR |
|---|---|---|
| 1 | ---- | 154.4 *s* |
| 2 | ---- | 112.5 *s* |
| 3 | ---- | 154.4 *s* |
| 4 | 6.22 *s* | 108.1 *d* |
| 5 | ---- | 142.2*s* |
| 6 | 6.22 *s* | 108.1 *d* |
| 1' | 2.58 *t* (*J* = 8.0 Hz) | 22.8*t* |
| 2' | 1.51 *m* | 31.4*t* |
| 3' | 1.40 *m* | 22.7*t* |
| 4' | 0.93 *t* (*J* = 7.0 Hz) | 13.9*q* |
| 1" | 2.44 *t* (*J* = 8.0 Hz) | 35.5*t* |
| 2" | 1.51 *m* | 30.7*t* |
| 3" | 1.51 *m* | 31.5*t* |
| 4" | 1.31 *m* | 22.5*t* |
| 5" | 0.88 *t* (*J* = 7.0 Hz) | 13.9*q* |

### Stempholone A

The stempholone A was isolated from the crude extract as a brown amorphous solid. Its molecular formula was determined as C₁₅H₂₆O₃ ([M]⁺,*m*/*z* 254.1888) (calcd. 254.1882) by high resolution mass spectrometry. The IR spectrum showed absorption bands at 3443, 1673, and 1650 cm⁻¹ attributable to hydroxyl groups, carbonyl groups and double bonds, respectively.

In the spectrum of ¹H-NMR, signals of two aliphatic chains, which integrated a total of twenty protons, a methylene group at δ 2.47 (1H, *dd, J* = 18.0 Hz, *J* = 10 Hz, H-4ax) and δ 2.61 (1H, *dd, J* = 18.0Hz, *J* = 6.0 Hz, H-4EC), and a methine bonded to an oxygen atom at δ 3.98 (1H, *dd, J* = 10.0 Hz, *J* = 6.0 Hz, H-5) were observed. Also, the signal of an olefinic proton at δ 5.89 (1H, *bs,* H-2) was observed at a low field, suggesting the presence of a tri-substituted double bond. The spectrum of 2D COSY ¹H NMR showed, as in the case of stemphol, the presence of aliphatic fragments butyl and pentyl which were determined by the correlations observed in the spin systems between H-1"-H₃-4" and H-1'- H₃-5', respectively. Another set of correlations that appeared in this spectrum were consistent with a -CH=C-CH₂-CH-(O)- pool. Thus, the geminal system at δ 2.47 (H-4'ax) and δ 2.61 (H-4 ec) showed a coupling with the signals at δ 3.98 (H-5) and δ 5.89 (H-2).

The ¹³C NMR spectrum showed the presence of fifteen signals, which were assigned to two methyls, eight methylenes, two methines and three quaternary carbons, according to an HSQC experiment. The resonances at δ 73.9 (C-5) and δ 79.6 (C-6) indicated the existence of two carbon atoms bonded to oxygen. In this spectrum, chemical shifts to 122.5 (C-2) and 164.2 (C-3), due to carbons of a double bond bound to an electron acceptor group, and the signal at δ 201.3 (C-1) of a carbonyl group stood out.

The location of the different functional groups was established based on the correlations shown in the HMBC experiment. Thus, connectivities between H-2 (δ 5.89), H-4 (δ 2.47 and 2.61 δ) and H-5 (δ 3.98) with C-6 (δ 79.6), of H-1" (δ 1.95) with C-1 (δ 200.1) and C-6 (δ 79.6), and of H-1' (δ 2.23) with C-2 (δ 122.5) and C-3 (δ 164.5) were observed.

The correlations of the HMBC experiments and 2D COSY ¹H NMR suggested the presence of a 6-butyl-3-pentylcyclohexene skeleton. The stereochemistry of C-5 and C-6 was deducted by NOE effects observed in the irradiation of H-4ax with H-1" and of H-4ec with H-5. The relative position of the alkyl chains butyl and pentyl was confirmed by NOE effects observed between H-2 and H-1', and H-4 and H-1' protons. These spectroscopic data allowed determining the structure of a compound as 6-butyl-5,6-dihydroxy-3-pentylcyclohex-2-en-1-one, not previously described in the literature, and which was named as stempholone A.
Stempholone A. [α]_{D} +4.4 (c 0.08, CHCl₃); IR (film) vₘₐₓ3443, 2957, 2930, 2861, 1673, 1650, 1626, 1467, 1378, 1257, 1142, 1075 cm⁻¹; for ¹H NMR data (CDCl₃, 500 MHz) see Table 2; for ¹³C NMR data (CDCl₃, 125 MHz) see Table 2; HREIMS m/z 254.1888 [M]⁺ (calculated for C₁₅H₂₆O₃, 254.1882); EIMS 70 eV m/z (rel. int.): 254 [M⁺] (5), 198 [M-C₄H₈]⁺ (35), 179 (8), 169 [M-C₄H₈-C₂H₅]⁺ (33), 151 (21), 139 [M+H-C₆H₁₂O₂]⁺ retro-Diels Alder fragmentation (100), 124 (22), 116 (58), 95 (20), 85 (54), 74 (55).

### Stempholone B

The stempholone B was isolated from the crude extract as a brown amorphous solid. The molecular formula was determined as C₁₅H₂₆O₄ from the molecular ion at 270.1833 *m*/*z* (calcd. 270.1831) in the mass spectrum of high resolution. The absorptions of the infrared spectrum at 3418, 1673, 1651 cm⁻¹ indicated the presence of hydroxyl groups, a carbonyl group, and double bonds, respectively. In the ¹H NMR spectrum, signals similar to those obtained for stempholone A were observed. The most significant difference between the two spectra was the presence of a signal at δ3.81 (1H, *td, J* = 12.0 Hz, *J* = 6.0 Hz, H-4'), which suggested the presence of an additional hydroxyl group that could be located at C-3 or C-4. The correlation observed in the HMBC experiment between the methyl groups in δ1.19 (3H, d, H-5') and the signal at δ 67.7 confirms the position of the hydroxyl group at C-4. Furthermore, the NOE effect between H-1 and H-4, as well as the coupling constants of H-3 allowed to determine the relative stereochemistry at C-2 and C-3. Based on the above data, the structure of this compound was assigned as 6-butyl-5,6-dihydroxy-3-(4-hydroxypentyl)cyclohex-2-en-1-one. It is the first time that this compound is isolated as a natural product and was named as stempholone B.
Stempholone B. [α]_{D} +12.5 (c 0,056, CHCl₃); IR (film) vₘₐₓ 3418, 295, 2931, 2872, 1673, 1667.1651, 1626, 1433, 1377, 1260, 1138, 1076 cm⁻¹; for ¹H NMR data (CDCl₃, 500 MHz) see Table 2; for ¹³C NMR data (CDCl₃, 125 MHz) see Table 2; HREIMS m/z 270.1833 [M]⁺ (calculated for C₁₅H₂₆O₄, 270.1831); EIMS 70 eV m/z (rel. int.): 270 [M⁺] (7), 252 [M-H₂O]⁺ (14), 197 [M+H-H₂O-2C₂H₄]⁺ (35), 179 [M+H-2H₂O-2C₂H₄]⁺ (12), 167 [M-H₂O-2C₂H₄-C₄H₉]⁺ (33), 155 (17),149 (26), 137 (21), 125 (10), 116 [M-C₁₁H₁₈O₃]⁺ retro-Diels Alder fragmentation (100), 109(28), 95 (54), 85 (85), 74 (96).

**Table 2. Spectroscopic data from ¹H NMR and ¹³C NMR of the stempholone A and B.**

| | **Stempholone A** | | **Stempholone B** | |
|---|---|---|---|---|
| H/C | ¹H NMR | ¹³C NMR | ¹H NMR | ¹³C NMR |
| 1 | ---- | 201.3 *s* | ---- | 201.3 *s* |
| 2 | 5.89*bs* | 122.5 *d* | 5.89 *bs* | 122.6 *d* |
| 3 | ---- | 164.2 *s* | ---- | 163.6 *s* |
| 4 | 2.61 *dd* (*J* = 18.0, 6.0 Hz) | 36.1 *t* | 2.63 *dd* (*J* = 19.0, 6.0 Hz) | 36.0 *t* |
| | 2.47 *dd* (*J* =18.0, 10.0 Hz) | | 2.47 *dd* (*J* =19.0, 10.0Hz) | |
| 5 | 3.98 *dd* (*J* = 10.0, 6.0 Hz) | 73.9 *d* | 3.98 *dd* (*J* = 10.0, 6.0 Hz) | 73.8 *d* |
| 6 | ---- | 79.6 *s* | ---- | 79.6 *s* |
| 1' | 2.23 *dd* (*J* = 7.0, 3.0 Hz) | 37.7 *t* | 2.23 *m* | 37.6 *t* |
| 2' | 1.45 *m* | 26.7 *t* | 1.45 *m* | 23.1 *t* |
| 3' | 1.30 *m* | 31.3 *t* | 1.30 *m* | 38.6 *t* |
| 4' | 1.28 *m* | 22.4 *t* | 3.81 *td* (*J* = 12.0, 6.0 Hz) | 67.7 *d* |
| 5' | 0.88 *t* (*J* = 7.0 Hz) | 13.9 *q* | 1.19 *d* (*J* = 6.0 Hz) | 23.8 *q* |
| 1" | 1.95 *dd* (*J* = 13.0, 6.0 Hz) | 29.3 *t* | 1.95 *dd* (*J* = 10.0, 6.0 Hz) | 29.3 *t* |
| | 1.45 *m* | | 1.45 *m* | |
| 2" | 1.28 *m* | 24.5 *t* | 1.28 *m* | 24.5 *t* |
| 3" | 1.32 *m* | 23.1 *t* | 1.28 *m* | 23.2 *t* |
| 4" | 0.85 *t* (*J* = 7.0 Hz) | 13.9 *q* | 0.85 *t* (*J* = 7.0 Hz) | 13.9 *q* |

### B) BIOCIDAL ACTIVITY

### Example 4. Activity against insect-pests

Breeding and maintenance of the insects was carried out in a temperature-controlled chamber at 24 ± 1 °C, 60-70 % relative humidity and a photoperiod of 16:8 hours (light: darkness). Larvae of *S*. *littoralis* were maintained on a semisynthetic diet (Poitut and Bues. 1970. Ann. Zool. Ecol. Anim. 2, 79-91) and aphids *M*. *persicae and Rhopalosiphum padi* on their host plants, pepper *-Capsicum annuum* L.- and barley *-Hordeum vulgare* L.-respectively. Tests on anti-feeding activity were carried out with newly hatched larvae of the sixth stage of *S*. *littoralis* and wingless adult aphids. The upper surface of leaf disks (1.0 cm²) of the pepper (*Capsicum annuum* L.) were treated with 10 µl of a solution (10 mg/ml for extracts and 5 mg/ml for pure products). Each test consisted of 5 *Petri* dishes with two (*S. littoralis*) larvae per dish or twenty boxes (2x2 cm) with ten *M. persicae or R. padi* aphids incubated in a growth chamber under the same conditions described for the breeding of the insects. Once consumed 75% of the surface of the control dishes (*S. littoralis*) or after 24 h (*M. persicae or R. padi*) the consumption (% FI) or settlement index (% SI), was calculated respectively. % FI = [1 - (T / C) x 100], where T and C are the consumption of treated leaves discs and control; % SI = [1 - (% T / C%)] where % C and % T are the percentage of aphids settled on the discs of control and treated leaves (Burgueño et al. 2008. J. Chem. Ecol. 34, 766-771). Compounds with an FI / SI> 70 % were tested in a dose - response experiment to calculate its relative potency (EC₅₀, being the effective dose for a reduction of 50 % of feeding).

**Table 3. Activity against insect-pests of the crude extract (100 µg/cm²), of the fractions H1-H8 [100 µg/cm²] and of the compounds with biocidal activity (50 µg/cm²).**

| Crude extract/fraction/compound | *S. littoralis* | *M. persicae* | *R. padi* |
|---|---|---|---|
| | % FI | % SI | |
| Crude extract | 97.21 ± 1.58 | 97.23 ± 1.27 | 40.02 ± 6.79 |
| H1 | 57.70 ± 8.81 | 50.29 ± 10.21 | 55.16 ± 6.27 |
| H2 | 65.22 ± 9.64 | 92.76 ± 2.59 | 72.57 ± 6.20 |
| H3 | 34.54 ± 11.70 | 91.51 ± 3.28 | 86.57 ± 3.11 |
| H4 | 32.83 ± 16.81 | 83.91 ± 4.06 | 38.17 ± 7.70 |
| H5 | 54.27 ± 11.78 | 47.54 ± 10.06 | 68.36 ± 6.41 |
| H6 | 78.51 ± 9.56 | 74.88 ± 6.77 | 34.29 ± 6.90 |
| H7 | 98.22 ± 1.44 | 93.18 ± 2.15 | 95.46 ± 2.11 |
| H8 | 79.98 ± 7.93 | 72.04 ± 5.65 | 59.68 ± 6.51 |
| **Stemphol** | 60.44 ± 8.7 | 85.35 ± 5.36 0.05 (0.01-0.301)* | 72.41 ± 5.61 |
| **Stempholone A** | 41.8 ± 7.9 | 81.42±5.61 0.15 (0.06-0.36)* | na |
| **Stempholone B** | 52.9 ± 5.9 | 83.87±6.89 0.02 (0.003-0.15)* | 45.13 ± 9.09 |

| | | | |
|---|---|---|---|
| *EC50 (µg/cm²) | | | |

### Example 5. Antifungal activity

Phytopathogenic fungi *Fusarium moniliforme* (Sheldon) [CECT 2152] *F*. *oxysporum fs. lycopersici* (Escalda) [CECT 2715] and *F. solani* (Mart) [CECT 2199] come from the Spanish Type Culture Collection (CECT). The strain *Botrytis cinerea Pers.:Fr.* (B05.10) is a donation from the Biochemistry Department at the University of La Laguna (ULL). For their maintenance the strains were grown on solid commercial medium PDA at 25 °C (*Fusarium*) or room temperature (*Botrytis*), and subsequent storage at -30 °C in vials with 18 % glycerol. To determine the antifungal activity, the agar dilution method was used (Murabayashi et al. 1991. J. Pesticide Sci. 16, 419-427). Samples of crude extract obtained as in Example 2 were incorporated into the culture medium (5 ml) at 5 different concentrations (1, 0.5, 0.1, 0.05 and 0.01 mg/ml). In parallel, controls were prepared with ethanol at a concentration of 2 %. The inoculation of the target organisms was carried out by stabbing (*Fusarium*) or with 5 mm diameter discs (*B. cinerea*)*.* Colonies grown in petri dishes and incubated for 48 h were digitized and measured using the ImageJ 1.43 software. The percentage of inhibition (% I) was calculated as: % I = (C-T/C) x 100, where C is the diameter of control colonies and T of the colonies of the tested samples. The growth inhibition effective dose (EC₅₀) was determined by linear regression analysis (% of inhibition of dose log).

**Table 4. Antifungal activity of the crude extract, of fractions H1-H8 and of the compounds (using 0.5 m/ml.) ^{a}Tested at 0.1 mg/ml.**

| Crude extract/fraction/compound | Antifungal activity (0.5 mg/ml) | | | |
|---|---|---|---|---|
| | EC₅₀ mg/ml (95 % CL) | | | |
| | *Fusarium oxysporum* | *F. moniliforme* | *F. solani* | *Botrytis cinerea* |
| Crude extract | 85.5±0.89 | 80.921±1.674 | 77.78±1.09 | 61.176±3.793 |
| | **0.144(0.113- 0.183)** | **0.196 (0.195- 0.196)** | **0.08(0.07-0.08)** | **0.30 (0.31- 0.30)** |
| H1 | 10.90±2.59 | 3.52±0.78 | 1.126±1.625 | 9.61±3.66 |
| H2 | 50.44±9.40 0.464(0.069-0.952) | 32.79±1.72 | 46.79±6.89 | 9.44±3.77^{a} |
| H3 | 69.38±1.17 0,179 (0.147-0.218) | 56.26±1.69^{a} | 82.55±2.9^{a} | 54.82±1.97^{a} |
| H4 | 3.61±1.18 | 0 | 15.262±2.782 | 1.03±1.13 |
| H5 | 12.79±1.84 | 12.87±2.86 | 15.328±3.006 | 16.03±2.30 |
| H6 | 22.68±2.62 | 22.93±3.48 | 36.459±3.545 | 22.83±2.72 |
| H7 | 0 | 0 | 4.629±1.937^{a} | 0.24±2.67 |
| H8 | 0.23±1.65 | 0 | 4.585±2.432 | 3.87±2.81 |
| **Stemphol** | 70.22±2.04 | 67.35±2.05 | 47.093±3.578 | 44.74±1.35 |
| | **0.01 (0.005- 0.024)** | **0.02 (0,020-0,021)** | | **0.53 (0.53- 0.54)** |
| **Stempholone A2** | Na | na | Na | **56.21±1.50** |
| | | | | **0.43 (0.42- 0.43)** |
| **Stempholone B** | ----- | 4.20±1.14^{a} | 49.94±4.40^{a} | 11.88±3.67 |
| | | | **0.21 (0.21-0.22)** | |

### Example 6. Nematicidal activity

The nematode population (*M. javanica*) was maintained in growth chambers on tomato plants *-Lycopersicon esculentum* (var Marmande)- at 25 °C and a relative humidity of 70 %. The tests were carried out according to the methodology described for *M. javanica* (Andres et al. 2012. Phytochem. Rev. 11, 371-390) using the biological stage of infective juveniles (J2). The activity of the dry crude extract obtained as in Example 2, their fractions and the compounds with biocidal activity was quantified at a final concentration per well of 1.0, 0.5 and/or 0.25 mg/ml, respectively. Each treatment was repeated four times and nematicidal activity was determined from the percentage of infective juveniles dead after 72h. In cases in which a mortality rate of > 99 % was determined, dose-response experiments were carried out to determine LC₅₀ and LC₉₀.

**Table 5. Nematicidal activity of the crude extract, of the fractions H1-H8 and of the compounds.**

| Crude extract/fraction/ compound | mg/ml | *M. javanica* % paralysed |
|---|---|---|
| Crude extract | 1 | **94.82 ± 0.75** |
| H1 | 1 | 0.41 ± 1.60 |
| H2 | 1 | 7.46 ± 1.79 |
| H3 | 1 | 0.00 ± 0.00 |
| H4 | 1 | 0.00 ± 0.00 |
| H5 | 1 | 10.39 ± 1.87 |
| H6 | 1 | 11.14 ± 3.23 |
| H7 | 1 | 0.98 ± 1.42 |
| H8 | 1 | 1.80 ± 0.65 |
| **Stemphol** | 0.5 | 1.24 ± 0.68 |
| **Stempholone A** | 0.5 | 83.05 ± 3.15 |
| | 0.25 | 2.8 ± 0.5 |
| **Stempholone B** | 0.5 | 1.24 ± 0.9 |

### Example 7: Phytotoxicity test

The phytotoxic activity of the crude extract, fractions and pure compounds (obtained according to Example 2 and 3) was evaluated against seeds of *Lactuca sativa* Teresa (Fito, Spain)

Experiments were carried out in 12-well plates (Falcon), using 20 µl (10 µg/µl) on paper discs of 2.5 cm in diameter positioned at the bottom of each well. 500 µl of distilled water and 10/5 seeds, were added to each well, and the plates were incubated in a growth chamber at 25 °C, 70 % relative humidity and a 16:08 L: D photoperiod. Seeds' germination was recorded for six days and the elongation of roots was assessed at the end of the experiment. The data were analysed by variance analysis (ANOVA). As positive control of germination inhibition juglone (5 µg/µl:germination lower than 5 %) was used.

The results are summarised in Table 6.

**Table 6. Phytotoxic effects of the extracts and fractions of the fungus Aa22 (100 µg/µl) and compounds (5 µg/µl) in Lactuca sativa**

| **Sample** | **Germination (%C)** | **Growth (%C)** |
|---|---|---|
| | **72h** | **Root** |
| **Aa22** | | |
| **complete extract** | 100 ± 0.00 | 221.64 ± 19.78 |
| **H1** | 100 ± 0.00 | 104.77 ± 8.74 |
| **H4** | 100 ± 0.00 | 133.66 ± 13.09 |
| **H5** | 100 ± 0.00 | 137.41 ± 13.10 |
| **H6** | 102.63 ± 4.08 | 246.57 ± 13.61 |
| **H7** | 100 ± 0.00 | 197.65 ± 16.18 |
| **H8** | 100 ± 0.00 | 104.77 ± 9.29 |
| **II** | 100 ± 0.00 | 140.73 ± 13.16 |
| **Ia** | 97.50 ± 3.54 | 230.26 ± 21.33 |
| **Ib** | 100 ± 0.00 | 109.26 ± 9.57 |

Tests show absence of phytotoxic effects of the extract, the fractions and the compounds la, Ib and II on germination and root development of the plant *Lactuca sativa. L. sativa* (lettuce), which is used as a model of dicot plant in phytotoxicity tests.

For reasons of completeness, various aspects of the invention are set out in the following numbered clauses:
Clause 1.- A fermentation product of an endophytic fungus of the species *Stemphylium solani* with broad-spectrum biocidal activity, characterised in that it comprises a compound of formula (I): 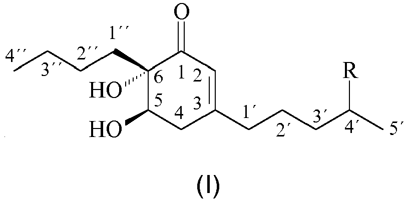 where R is selected from H and OH,
   or an isomer, or a salt or a solvate thereof.
Clause 2.- The fermentation product, according to Clause 1, characterised in that it comprises simultaneously a compound of formula (I) wherein R is H, and another compound of formula (I) wherein R is OH, or an isomer, or a salt or a solvate thereof.
Clause 3.- The fermentation product according to any one of Clauses 1 and 2, characterised in that it further comprises another compound with broad-spectrum biocidal activity of formula (II): 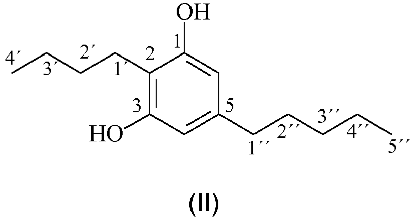 or an isomer, or a salt or a solvate thereof.
Clause 4.- A compound of formula (I): 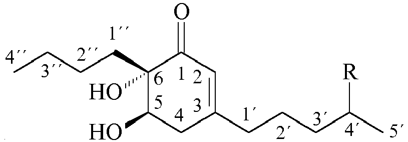 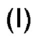 where R is selected from H and OH,
   or an isomer, or a salt or a solvate thereof.
Clause 5.- A process for preparing the fermentation product defined according to any one of Clauses 1 to 3, characterised in that it comprises culturing mycelium of an endophytic fungus of the species *Stemphylium solani* in a culture medium.
Clause 6.- The process for preparing according to Clause 5, characterised in that it further comprises a drying step using a technique selected from extraction with organic solvent with previous filtration or lyophilization.
Clause 7.- The process according to Clause 6, characterised in that the endophytic fungus is the strain Aa22 of *Stemphylium solani* with deposit number CECT 20941.
Clause 8.- Use of a fermentation product as defined according to Clauses 1 to 3, or a compound of general formula (I) as defined in Clause 4, or a compound of formula (II) as defined in Clause 3 for developing a broad-spectrum biocidal composition.
Clause 9.- A broad-spectrum biocidal composition comprising a fermentation product as defined according to Clauses 1 to 3, or a compound of general formula (I) as defined in Clause 4, or a compound of formula (II) as defined in Clause 3.
Clause 10.- Use of the biocidal composition according to Clause 9 as broad-spectrum biocidal agent for simultaneous control of more than one category of harmful organisms affecting plants.
Clause 11.- Use according to Clause 10, characterised in that the categories of harmful organisms affecting plants are selected from insect-pests, fungi and nematodes.
Clause 12.- Use according to any one of Clauses 10 and 11, characterised in that the categories of harmful organisms affecting plants are simultaneously insect-pests, fungi and nematodes.
Clause 13.- A method for the simultaneous control of more than one category of harmful organisms affecting plants comprising administering an effective dose of a fermentation product as defined according to Clauses 1 to 3, or of a compound of general formula (I) as defined in Clause 4, or of a compound of formula (II) as defined in Clause 3.
Clause 14.- The control method according to Clause 13, characterised in that the categories of harmful organisms affecting plants are selected from insect-pests, fungi and nematodes.
Clause 15.- The control method according to any of Clauses 13 and 14, characterised in that the categories of harmful organisms affecting plants are simultaneously insect-pests, fungi and nematodes.

## Claims

1. A fermentation product of a fermenting micro-organism comprising a compound of formula (I): 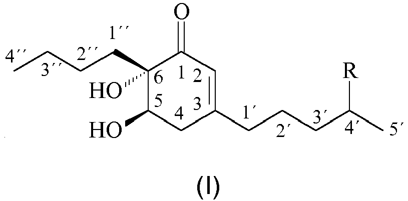 where R is selected from H and OH,
or an isomer, or a salt or a solvate thereof.

2. The fermentation product according to Claim 1, comprising a compound of formula (Ia), or an isomer, salt or solvate of (Ia), and a compound of formula (Ib), or an isomer, salt or solvate of (Ib):

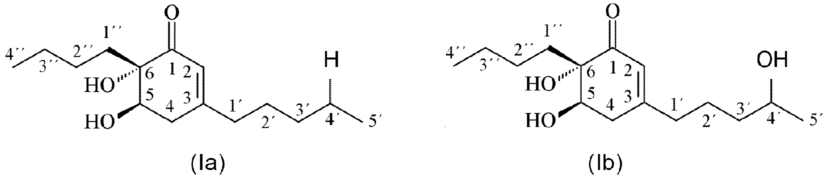

3. The fermentation product according to any one of the Claims 1 and 2, further comprising a compound of formula (II): 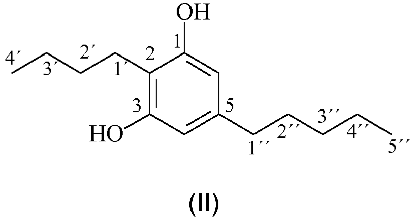 or an isomer, or a salt or a solvate thereof.

4. The fermentation product according to any one of the Claims 1-3, which is an extract.

5. The fermentation product according to any one of the Claims 1-4, which is free of the fermenting micro-organism.

6. The fermentation product according to any one of the Claims 1-4, comprising the fermenting micro-organism inactivated.

7. The fermentation product according to any one of the Claims 1-6, wherein the fermenting micro-organism is a fungus.

8. The fermentation product according to Claim 7, wherein the fungus is an endophytic fungus.

9. The fermentation product according to Claim 8, wherein the endophytic fungus is of the genus *Stemphylium.*

10. The fermentation product according to Claim 9, wherein the endophytic fungus is a strain of *Stemphylium solani.*

11. The fermentation product according to Claim 10, wherein the endophytic fungus is the strain of *Stemphylium solani* CECT 20941.

12. A compound of formula (I): 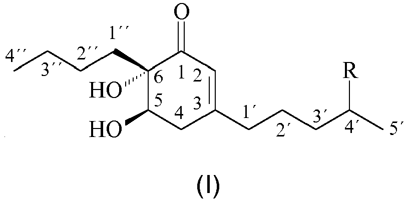 where R is selected from H and OH,
or an isomer, or a salt or a solvate thereof.

13. A process for preparing the fermentation product as defined in any one of the Claims 1 to 11, comprising (a) a fermentation step, which comprises cultivating the fermenting micro-organism in a culture medium, which results in the production of a compound of formula (I); and, optionally, (b) one or more extraction steps.

14. The process according to Claim 13, wherein the fermentation step is carried out in the presence of a cereal.

15. The process according to any one of the Claims 13 to 14, wherein the fermentation step is carried out in the dark.

16. The process according to any one of the Claims 13 to 15, wherein the fermentation step is carried out at room temperature.

17. The process according to any one of the Claims 13 to 16, wherein the step (b) of extraction of the fermentation product is carried out by decanting using a polar aprotic solvent or mixture of polar aprotic solvents.

18. The process according to any one of the Claims 13 to 17, comprising a step prior to step (a) wherein the fermenting micro-organism is grown in a culture medium.

19. The process according to any one of the Claims 13 to 18, further comprising a step (i.1) subsequent to the fermentation step (a), wherein the fermenting micro-organism is removed.

20. The process according to any one of the Claims 13 to 18, further comprising a step (i.2) subsequent to the fermentation step (a), wherein the fermenting micro-organism is inactivated.

21. The process according to any one of the Claims 13 to 20, comprising a step (c) of purification of the extract resulting from step (b), (i.1.) or (i.2.).

22. The process according to Claim 21, wherein the purification step (c) is carried out through chromatography.

23. The process according to any one of the Claims 13 to 22, further comprising a drying step of the extract resulting from step (b), (i.1.), (i.2.) and/or (c).

24. The process according to Claim 23, wherein the drying step of the extract comprises vacuum drying.

25. The process according to any one of the Claims 13 to 24, wherein the fermenting micro-organism is a fungus, preferably an endophytic fungus, preferably a strain of *Stemphylium,* preferably a strain of *Stemphylium solani,* preferably a strain of *Stemphylium solani* with deposit number CECT 20941.

26. A fermentation product comprising the compound of formula (I) as defined in Claim 12, obtainable by the process as defined in any one of Claims 13-25.

27. The fermentation product according to Claim 26, further comprising the compound of formula (II) as defined in Claim 3.

28. A process for preparing a compound of formula (I) as defined in Claim 12, comprising the step of isolation of the compound of formula (I) from the product resulting from step (b), (i.1.), (i.2) or (c), defined in Claims 13 to 24.

29. A strain isolated from the endophytic fungus of the species *Stemphylium solani* deposited in the Spanish Type Culture Collection with deposit number CECT20941 or a mutant thereof that maintains the ability to produce the compound of formula (I).

30. A biocidal composition comprising the fermentation product as defined in any of Claims 1 to 11, or the fermentation product as defined in any one of the Claims 26-27, or the compound of formula (I) as defined in Claim 12, or the compound of formula (II) as defined in Claim 3, or the strain of *Stemphylium solani* of Claim 29.

31. Use of a fermentation product as defined in any one of the Claims 1 to 11, or the fermentation product as defined in any one of the of Claims 26-27, or the compound of general formula (I) as defined in Claim 12, or the compound of formula (II) as defined in Claim 3, or the strain of *Stemphylium solani* of Claim 28 as a biocidal agent.

32. Use of a fermentation product as defined in any one of the Claims 1 to 11, or the fermentation product as defined in any one of the Claims 26-27, or the compound of general formula (I) as defined in Claim 12, or the compound of formula (II) as defined in Claim 3, or the strain of *Stemphylium solani* of Claim 29, or the biocidal composition as defined in Claim 30, for the simultaneous control of more than one category of harmful organisms affecting plants.

33. The use according to any one of Claims 31-32, wherein the categories of harmful organisms affecting plants are selected from insect-pests, fungi and nematodes.

34. The use according to any one of Claims 31-33, wherein the categories of harmful organisms are simultaneously insect-pests, fungi and nematodes.

35. A method for controlling harmful organisms affecting plants comprising administering an effective dose of the fermentation product as defined in any one of the Claims 1-11, or of the fermentation product as defined in any one of Claims 26-27, or of the compound of formula (I) as defined in Claim 12, or of the compound of formula (II) as defined in Claim 3, or of the strain of *Stemphylium solani* as defined in Claim 29, or of the biocidal composition as defined in Claim 30.

36. The method of Claim 35, which is simultaneously effective against more than one category of harmful organisms.

37. The control method according to any one of Claims 35-36, wherein the categories of harmful organisms affecting plants are selected from insect-pests, fungi and nematodes.

38. The control method according to any one of Claims 35-37, wherein the categories of harmful organisms are simultaneously insect-pests, fungi and nematodes.
